# EUROPÄISCHE PATENTSCHRIFT

(11) **EP 3 016 698 B1**
(45) Veröffentlichungstag und Bekanntmachung des Hinweises auf die Patenterteilung: **24.01.2018**
(21) Anmeldenummer: 15701466.3
(22) Anmeldetag: 09.01.2015
(51) Int. Cl.: A61M 5/145, A61M 5/38

(54) **DAUERINFUSIONSVORRICHTUNG**
CONTINUOUS INFUSION DEVICE
DISPOSITIF D'INFUSION DURÉE

(30) Priorität: 19.02.2014 DE 202014001525 U
(43) Veröffentlichungstag der Anmeldung: 11.05.2016
(73) Patentinhaber: H & B Electronic GmbH & Co. KG, 75392 Deckenpfronn (DE)
(72) Erfinder: MORLOK, Tobias, 71159 Mötzingen (DE); WEBER, Wilfried, 72296 Schopfloch (DE)
(74) Vertreter: Wacker, Jost Oliver
(86) Internationale Anmeldenummer: PCT/EP2015/000025
(87) Internationale Veröffentlichungsnummer: WO 2015/124247

(56) Entgegenhaltungen:
- WO-A1-2013/051115
- DE-A1- 2 410 868
- DE-U1-202013 000 411
- US-A- 4 059 110
- US-A1- 2005 148 868

## Beschreibung

Die Erfindung betrifft eine Vorrichtung für Dauerinfusionen, wie beispielsweise zur Verabreichung von Medikamenten gegen Multiple Sklerose, nach dem Oberbegriff des Anspruchs 1 sowie eine Infusionsanordnung mit einer solchen Vorrichtung. Die Dauerinfusionsvorrichtung weist dabei ein Gehäuse auf, in dem eine Infusionsgeräteaufnahme für die Aufnahme eines Infusionsgerätes mit wenigstens einem mittels eines Stößels auspressbaren Behälters vorgesehen ist und die sich entlang einer Haupterstreckungsachse von einem stößelseitigen Ende zu einem auslassseitigen Ende erstreckt. Zudem weist die Dauerinfusionsvorrichtung eine Betätigungsvorrichtung zum selbsttätigen Auspressen des Infusionsgerätes auf. Die Betätigungsvorrichtung weist wiederum ein Stößelanschlags/-betätigungs-Element auf, das mittels eines mechanischen Antriebes verlagert werden kann. Ferner ist eine Steuerungsanordnung vorgesehen, mittels der eine Vorschubgeschwindigkeit der Betätigungsvorrichtung eingestellt werden kann.

Mit derartigen Dauerinfusionsvorrichtungen können ortsunabhängig kleinste Infusionsströme eines Medikaments über mehrere Stunden hinweg kontinuierlich in die Blutbahn eines Patienten abgegeben werden. Dies ermöglicht einen sehr flexiblen und beispielsweise mobilen Einsatz der Vorrichtungen.

Aus US 4,059,110 ist eine Infusionsvorrichtung bekannt, mittels der über einen vorgegebenen Zeitraum eine vorbestimmte Menge eines flüssigen Medikamentes aus einer Spritze heraus über eine Infusionsleitung verabreicht werden kann. Die Infusionsvorrichtung weist hierfür ein Gehäuse zur Aufnahme der Spritze auf, in dem ein Beaufschlagungsarm zur Kraftbeaufschlagung des Spritzenstößels vorgesehen ist. Der Beaufschlagungsarm ist hierzu mittels eines Uhrwerks verlagerbar, über das die gewünschte Infusionszeit einstellbar ist.

Nachteilig an der bekannten Infusionsvorrichtung ist, dass vor dem Einbau der Spritze und der anschließenden Verabreichung des jeweiligen flüssigen Medikamentes zunächst eine Entlüftung der Spritze und der Infusionsleitung vorgenommen werden muss. Dies ist ein wesentlicher Grund dafür, dass diese Vorrichtung lediglich von geschultem Personal verwendet werden kann. Darüber hinaus weist die Vorrichtung insbesondere aufgrund des verwendeten Uhrwerks relativ hohe Herstellungskosten auf und ist sehr empfindlich gegenüber äußeren Einflüssen. Zudem unterliegt das Uhrwerk einem gewissen Verschleiß und muss in gewissen Zeitabständen nachgestellt werden, um eine exakte Funktionsweise sicherstellen zu können.

US 2005/0148868 A1 zeigt einen Injektor, dessen Gehäuse eine Unterseite ausbildet, die sich parallel zur Haupterstreckungsachse einer an einer Aufnahme gehaltenen Spritze erstreckt. Der Injektor ist dabei verschwenkbar an einem Befestigungsarm gehalten.

DE 2 410 868 A beschreibt eine Vorrichtung zum Injizieren von Kontrastmitteln mit einer fest eingebauten Spritzenanordnung. Die Spritzenanordnung ist dabei verdrehbar gegenüber einer Steueranordnung gehalten.

Die Aufgabe der Erfindung ist es, bei einer gattungsgemäßen Dauerinfusionsvorrichtung die genannten Nachteile zu vermeiden und auch eine Verwendung durch nicht medizinisch geschultes Personal und insbesondere durch die Patienten selbst zu ermöglichen.

Diese Aufgabe wird durch eine Dauerinfusionsvorrichtung mit den Merkmalen des Anspruchs 1 gelöst. Dabei weist die Haupterstreckungsachse der Infusionsgeräteaufnahme gegenüber einer horizontalen Lagerebene des Gehäuses einen festen vorgegebenen Anstellwinkel auf, wobei das auslassseitige Ende oberhalb des stößelseitigen Endes der Infusionsgeräteaufnahme angeordnet ist. Hierdurch gibt die Infusionsgeräteaufnahme eine Position des aufzunehmenden Infusionsgerätes vor, in der sich die im Infusionsgerät enthaltene Luft in einem vorbestimmten oberen Bereich ansammelt. Auf diese Weise wird sichergestellt, dass bei einer Beaufschlagung des Infusionsgerätes zunächst die Luft aus diesem beziehungsweise aus einer angeschlossenen Infusionsleitung entweicht. Hierdurch kann das in der Infusionsgeräteaufnahme aufzunehmende Infusionsgerät besonders einfach entlüftet werden, wodurch es wiederum möglich ist, dass die Entlüftung selbsttätig oder von medizinisch ungeschultem Personal oder von dem Patienten selbst vorgenommen werden kann.

In einer vorteilhaften Ausführungsform beträgt der Anstellwinkel dabei zwischen 5 und 90°, so dass auch bei leichter Unebenheit der jeweiligen Abstellfläche eine sichere Ansammlung der im Infusionsgerät enthaltenen Luft in einem vorbestimmten oberen Bereich gewährleistet werden kann.

Dabei ist es günstig, wenn die Infusionsgeräteaufnahme eine Codierung für eine vorbestimmte Winkelposition, das heißt einem Verdrehwinkel orthogonal zur Haupterstreckungsachse, des aufzunehmenden Infusionsgerätes bezüglich der Haupterstreckungsachse aufweist. Hierdurch kann insbesondere bei einem Infusionsgerät mit exzentrisch angeordnetem Auslass, wie beispielsweise einer Spritze mit außermittiger Spritzenöffnung, sichergestellt werden, dass der Auslass nach dem Einbau des Infusionsgerätes in die Infusionsgeräteaufnahme den obersten Bereich des Infusionsgerätes bezüglich der Gravitationsrichtung bildet und sich die enthaltene Luft an diesem ansammelt.

Vorteilhafterweise ist die Infusionsgeräteaufnahme mit dem stößelseitigen Ende in Richtung des Stößelanschlags/-betätigungs-Elementes verlagerbar und ist hierzu mit einem zwischen einer Schließstellung und einer Offenstellung verlagerbaren Stellglied bewegungsgekoppelt. Hierdurch ermöglicht die Dauerinfusionsvorrichtung in der besagten Offenstellung einen komfortablen Einbau beziehungsweise Ausbau des Infusionsgerätes bei beabstandetem Stößelanschlags/-betätigungs-Element.

Hierbei ist es günstig, wenn das Stellglied Teil einer Verlagerungsvorrichtung ist, die bei einer Schließbewegung eines Gehäusedeckels mit einer Antriebskraft beaufschlagbar ist und dadurch zur Verlagerung der Infusionsgeräteaufnahme zum Stößelanschlags/-betätigungs-Element dient. Dies ermöglicht eine einfache Bewegungskoppelung zwischen Gehäusedeckel und Infusionsgeräteaufnahme, so dass die Infusionsgeräteaufnahme durch bloßes Schließen des Deckels selbsttätig in eine Anschlagsposition am Stößelanschlags/-betätigungs-Element verbracht werden kann.

Vorteilhafterweise weist die Verlagerungsvorrichtung dabei eine Ausgleichsfeder auf, mittels der Toleranzen hinsichtlich der Abmessungen und der Füllstände des jeweils eingesetzten Infusionsgerätes ausgeglichen werden können.

In einer besonders vorteilhaften Ausführungsform der Dauerinfusionsvorrichtung ist der Antrieb im Wesentlichen durch eine Antriebsfeder gebildet. Hierdurch kann ein rein mechanisch arbeitender Antrieb zur Verfügung gestellt werden, mittels dem unabhängig von einem lokal vorhandenen Stromnetz oder einer Batteriespannung kleine Infusionsströme eines Medikaments über mehrere Stunden hinweg kontinuierlich in die Blutbahn eines Patienten abgegeben werden können. Dies ermöglicht einen sehr flexiblen und beispielsweise mobilen Einsatz der Vorrichtung, wobei auch keinerlei Vorsichtsmaßnahmen hinsichtlich eines möglichen Stromausfalls oder einer nicht ausreichenden Batteriespannung während eines Infusionsvorganges erforderlich sind.

In einer weiteren vorteilhaften Ausführungsform weist die Steuerungsanordnung eine separate Steuerungsantriebsfeder auf. Durch diese eigene Kraftspeicherung der Steuerungsanordnung ist es möglich, eine Rückkoppelung der Antriebsfeder auf die Steuerungsanordnung zu unterbinden, was einen besonders exakten und sicheren Betrieb der Dauerinfusionsvorrichtung ermöglicht.

Ferner ist es günstig, wenn zwischen dem Antrieb und der Steuerungsanordnung eine Selbsthemmung vorgesehen ist, die mittels eines seitens der Steuerungsanordnung generierbaren Drehmomentes überwindbar ist. Durch eine solche Selbsthemmung kann die Verlagerungsbewegung der Betätigungsvorrichtung seitens der Steuerungsanordnung sicher gestoppt und dadurch der jeweilige Infusionsvorgang zum vorgegebenen Zeitpunkt exakt beendet werden.

Vorteilhafterweise ist die Selbsthemmung dabei durch ein Schneckenradgetriebe gebildet. Das Schneckenradgetriebe ermöglicht hierbei mit einfachen Mitteln eine zuverlässige Hemmung der Verlagerungsbewegung der Betätigungsvorrichtung.

Zudem ist es günstig, wenn die Steuerungsanordnung einen einstellbaren Rotationsdämpfer aufweist, mittels dem die Vorschubgeschwindigkeit der Betätigungsvorrichtung begrenzt werden kann. Durch die Verwendung eines solchen Rotationsdämpfers ist die Infusionsgeschwindigkeit beziehungsweise die Infusionszeit besonders exakt und stufenlos einstellbar.

Ferner wird die oben genannte Aufgabe durch eine Infusionsanordnung gelöst, die eine Dauerinfusionsvorrichtung in einer der oben beschriebenen Ausführungsformen und ein Infusionsgerät aufweist, wobei das Infusionsgerät durch eine in Größe und Geometrie auf die Infusionsgeräteaufnahme abgestimmte Infusionsspritze mit einem exzentrisch angeordneten Spritzenauslass gebildet ist. Hierdurch kann sichergestellt werden, dass sich die in dem Infusionsgerät enthaltene Luft im eingebauten Zustand an dem oben angeordneten Spritzenauslass ansammelt, was eine besonders einfache beziehungsweise selbsttätige Entlüftung ermöglicht.

In einer besonders günstigen Ausführungsform der Infusionsanordnung ist die Infusionsspritze dabei mit einer Infusionsleitung verbunden, an deren Ende eine zur Entlüftung dienende hydrophobe Membran vorgesehen ist, die luftdurchlässig ist aber flüssigkeitsabdichtend wirkt. Nach der Verbindung der Infusionsleitung mit dem an oberster Stelle des Infusionsgerätes angeordneten Spritzenauslass kann somit sowohl dieser als auch die Infusionsleitung durch bloße Beaufschlagung des Spritzenstößels entlüftet werden.

Vorteilhafterweise ist die in der Infusionsgeräteaufnahme aufgenommene Infusionsspritze dabei durch die Schließbewegung des Gehäusedeckels oder der Verlagerungsvorrichtung soweit gegen das Stößelanschlags/-betätigungs-Element verlagerbar, dass die hydrophobe Membran durch die im Behälter aufgenommene Infusionsflüssigkeit über die Infusionsleitung beaufschlagt wird. Auf diese Weise kann durch das alleinige Schließen des Deckels der Dauerinfusionsvorrichtung die selbsttätige Entlüftung sowohl des Infusionsgerätes als auch der Infusionsleitung sichergestellt werden.

Zudem ist es günstig, wenn der hydrophoben Membran eine weitere hydrophobe Membran vorgeschaltet ist, die ab einem vorbestimmten Schwellendruck hinsichtlich der Infusionsflüssigkeit durchlässig ist. Durch diese vorgeschaltete Membran, die gegenüber der anderen Membran leichter durchlässig ist, ist auch nach der vollständigen Entlüftung noch eine zumindest geringe Verlagerung des Infusionsgerätes gegenüber der Betätigungsvorrichtung möglich, um beispielsweise die Festlegung der Infusionsgeräteaufnahme in einer von mehreren Rastpositionen zu ermöglichen.

Vorteilhafterweise ist eine Verriegelungsanordnung vorgesehen, mittels der die Infusionsgeräteaufnahme gegenüber dem Gehäuse festlegbar ist, wobei die Verriegelungsanordnung durch einen an der Infusionsgeräteaufnahme angreifenden Bewegungswiderstand aktivierbar ist, der bei der Verlagerung des Infusionsgerätes gegen das Stößelanschlags/-betätigungs-Element durch den sich zwischen der hydrophoben Membran und dem Spritzenstößel aufbauenden Flüssigkeitsdruck generiert wird. Hierdurch kann die Verriegelung der Infusionsgeräteaufnahme jeweils bei Erreichen einer entlüfteten Ausgangsstellung selbsttätig erfolgen.

Hierbei ist es günstig, wenn die Verriegelungsanordnung ein Keilgetriebe aufweist, mittels dem wenigstens ein an der Infusionsgeräteaufnahme mitgeführtes Verriegelungselement in eine gehäuseseitige Verriegelungsaufnahme verbringbar ist. Hierdurch ist mit einfachen Mitteln eine stabile selbsttätige Verriegelung der Infusionsgeräteaufnahme möglich.

In den Figuren ist eine beispielhafte Ausführungsform der Erfindung dargestellt. Es zeigen:
- Figur 1: eine perspektivische Ansicht einer erfindungsgemäßen Dauerinfusionsvorrichtung mit einem Gehäusedeckel in Offenstellung,
- Figur 2: eine Draufsicht auf eine Infusionsgeräteaufnahme der Dauerinfusionsvorrichtung mit darin aufgenommenem Infusionsgerät,
- Figur 3: einen Schnitt durch die Infusionsgeräteaufnahme in Ebene III-III aus Fig. 2,
- Figur 4: eine perspektivische Ansicht einer getrennt dargestellten Verlagerungsvorrichtung der Dauerinfusionsvorrichtung nach Fig. 1,
- Figur 5A: einen Schnitt durch die Infusionsgeräteaufnahme in Ebene V-V aus Fig. 2 in einer Passivstellung einer Verriegelungsanordnung,
- Figur 5B: einen Schnitt durch die Infusionsgeräteaufnahme in einer Aktivstellung der Verriegelungsanordnung nach Fig. 5A und
- Figur 6: eine perspektivische Ansicht einer getrennt dargestellten Steuerungsanordnung der Dauerinfusionsvorrichtung nach Fig. 1.

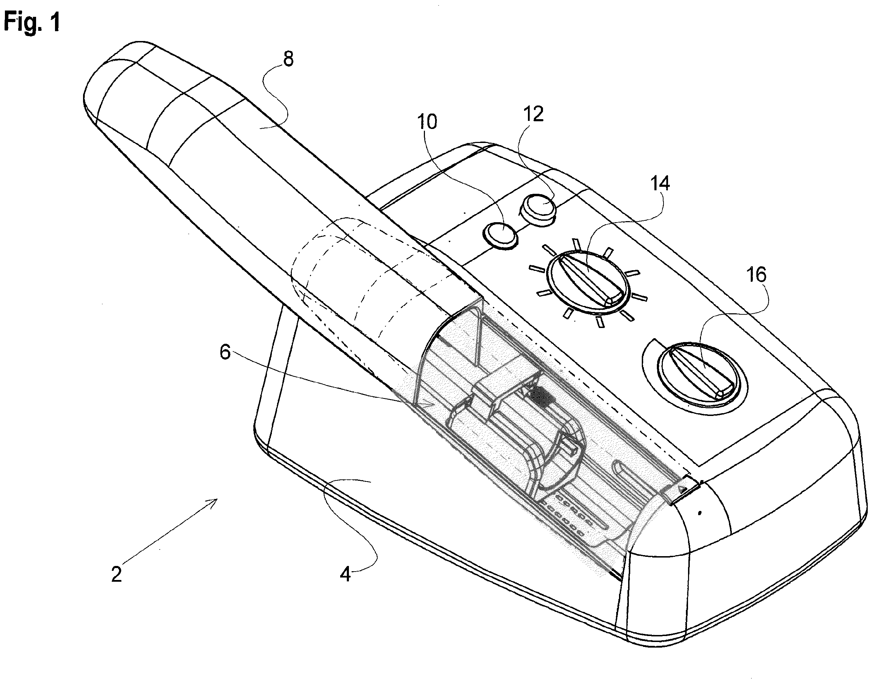

Fig. 1 zeigt eine Dauerinfusionsvorrichtung 2, die zur Verabreichung von kleinen Infusionsströmen einer Infusionsflüssigkeit, die beispielhaft durch ein flüssiges Medikament gegen Multiple Sklerose gebildet ist, über einen ausgedehnten Zeitraum von mehreren Minuten oder mehreren Stunden dient.

Die Dauerinfusionsvorrichtung 2 weist ein Gehäuse 4 auf, an dem eine verschiebbare Infusionsgeräteaufnahme 6 vorgesehen ist. Ferner ist an dem Gehäuse 4 ein Gehäusedeckel 8 vorgesehen, mittels dem der Zugriff auf die Infusionsgeräteaufnahme 6 verschlossen oder freigegeben werden kann. Hierzu ist der Gehäusedeckel 8 zwischen einer dargestellten Offenstellung und einer durch strichpunktierte Linien dargestellte Schließstellung verlagerbar.

Ferner sind an dem Gehäuse mehrere Bedienelemente vorgesehen, über die der Benutzer an der Dauerinfusionsvorrichtung 2 einen gewünschten Infusionsablauf einstellen sowie starten und stoppen kann. Die Bedienelemente bestehen dabei beispielsweise aus einem ersten Druckknopf 10 zum Starten und einem zweiten Druckknopf 12 zum Stoppen eines Infusionsvorganges sowie aus einem ersten Drehschalter 14 zur Einstellung eines bestimmten Infusionsvolumens und einem zweiten Drehschalter 16 zur Einstellung einer bestimmten Infusionsgeschwindigkeit.

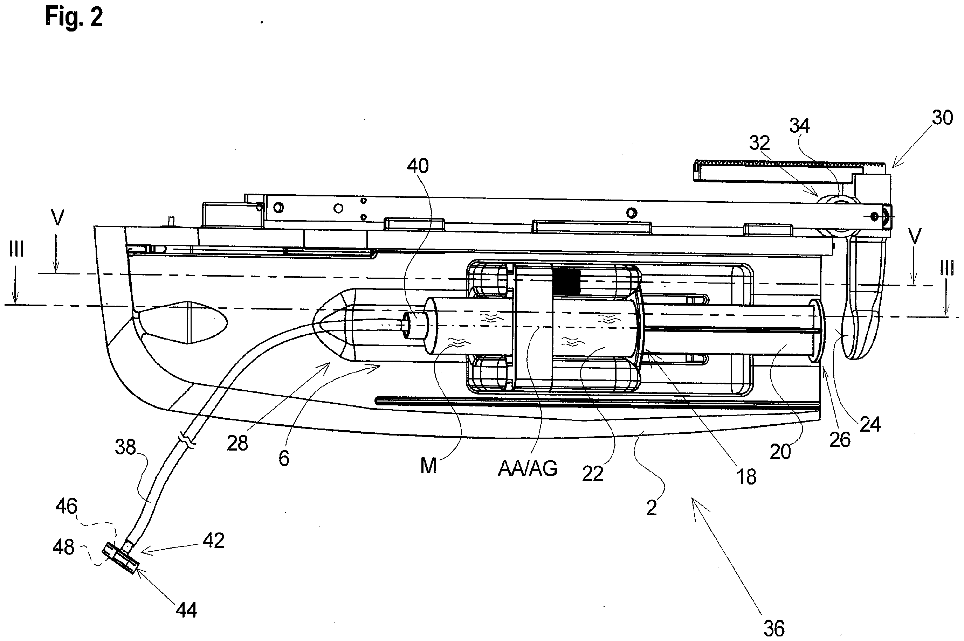

Wie aus Fig. 2 zu entnehmen ist, dient die Infusionsgeräteaufnahme 6 zur Aufnahme eines Infusionsgerätes 18 in Form einer Spritze, das einen mittels eines Stößels 20 auspressbaren Behälter 22 aufweist, in dem eine zu verabreichende Infusionsflüssigkeit in Form eines flüssigen Medikamentes M aufgenommen ist.

Wie aus Fig. 2 ferner zu entnehmen ist, ist die verlagerbare Infusionsgeräteaufnahme 6 in der dargestellten Offenstellung derart angeordnet, dass das Infusionsgerät 18 auch bei vollständig herausgezogenem Stößel 20 beabstandet zu einem Stößelanschlags/-betätigungs-Element 24 aufgenommen werden kann. Hierbei erstreckt sich die Infusionsgeräteaufnahme 6 von einem stößelseitigen Ende 26 zu einem auslassseitigen Ende 28 entlang einer Haupterstreckungsachse AA, die im eingebauten Zustand des Infusionsgerätes 18 mit dessen Haupterstreckungsachse AG übereinstimmt.

Das Stößelanschlags/-betätigungs-Element 24 ist Teil einer Betätigungsvorrichtung 30, mittels der das Infusionsgerät 18 selbsttätig ausgepresst werden kann. Hierzu weist die Betätigungsvorrichtung 30 einen Antrieb 32 auf, der im Wesentlichen durch eine Antriebsfeder 34 gebildet ist, mittels der das Stößelanschlags/- betätigungs-Element 24 gegen den Stößel 20 des Infusionsgerätes 18 gepresst werden kann.

Die Dauerinfusionsvorrichtung 2 bildet in dem in Fig. 2 dargestellten Zustand mit eingebautem Infusionsgerät 18 eine Infusionsanordnung 36, die ferner eine Infusionsleitung 38 aufweist, die mit einem am auslassseitigen Ende 28 der Infusionsgeräteaufnahme 6 angeordneten Spritzenauslass 40 des Infusionsgerätes 18 verbunden ist. Die Infusionsleitung 38 weist dabei an einem vom Infusionsgerät 18 abgewandten Abgabeende 42 eine Entlüftungseinrichtung 44 auf, in der eine erste hydrophobe Membran 46 und eine zweite hydrophobe Membran 48 vorgesehen sind. Beide hydrophobe Membrane 46, 48 wirken dabei grundsätzlich luftdurchlässig aber flüssigkeitsundurchlässig. Durch Aufbringung eines gewissen Flüssigkeitsdruckes kann diese Flüssigkeitsundurchlässigkeit jedoch aufgehoben werden, wobei im Falle der ersten Membran 46 hierfür ein geringerer Flüssigkeitsdruck notwendig ist als im Falle der zweiten Membran 48.

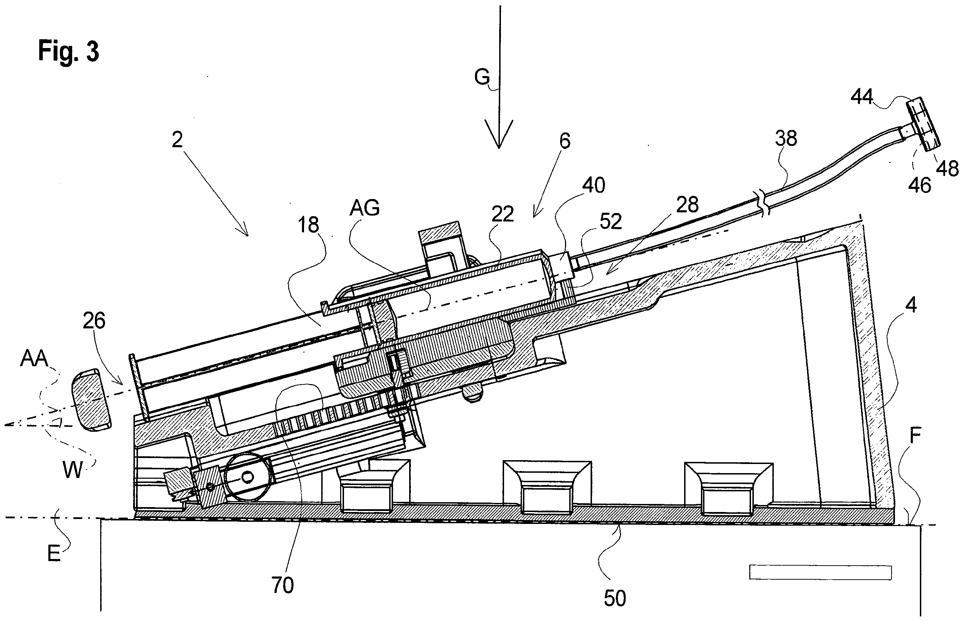

Wie insbesondere aus Fig. 3 zu entnehmen ist, weist die Haupterstreckungsachse AA der Infusionsgeräteaufnahme 6 einen Anstellwinkel W gegenüber einer Lagerebene E auf, der zwischen 5 und 90° liegt. Die Lagerebene E wird dabei durch einen bodenseitigen Kontaktbereich 50 des Gehäuses 4 aufgespannt, über den die Dauerinfusionsvorrichtung 2 auf einer vorzugsweise ebenen horizontalen Abstellfläche F abstellbar ist. Der Kontaktbereich 50 kann dabei, wie dargestellt, durch eine Lagerplatte oder alternativ auch beispielsweise durch mehrere beabstandete Kontaktflächen beziehungsweise Stellfüße gebildet sein. In jedem Fall ist dabei durch den vorgesehenen Anstellwinkel W zumindest bei im Wesentlichen horizontaler Ausrichtung der Lagerebene E das auslassseitige Ende 28 der Infusionsgeräteaufnahme 6 über dem stößelseitigen Ende 26 positioniert.

Wie aus Fig. 3 ferner zu entnehmen ist, ist der Spritzenauslass 40 des Infusionsgerätes 18 gegenüber dessen Haupterstreckungsachse AG beispielhaft exzentrisch ausgeführt. In diesem Fall sind am auslassseitigen Ende 28 Codierungsmittel 52 vorgesehen, die die Infusionsgeräteaufnahme 6 gegenüber dem Infusionsgerät 18 derart codieren, dass dieses gegenüber der Haupterstreckungsachse AA nur in der dargestellten Winkelposition in die Infusionsgeräteaufnahme 6 eingesetzt werden kann, in der der Spritzenauslass 40 bezüglich der Gravitationsrichtung G in maximal oberster Position angeordnet ist.

Hierdurch ist sichergestellt, dass sich im Behälter 22 enthaltene Luft des aufgenommenen Infusionsgerätes 18 am Spritzenauslass 40 ansammelt und sich bei Betätigung des Stößels 20 über die Infusionsleitung 38 vor der Fließfront des flüssigen Medikamentes weiter zur Entlüftungseinrichtung 44 verlagert, wo die Luft über die hydrophoben Membrane 46, 48 austreten kann. Der freie Fließquerschnitt der Infusionsleitung 38 ist hierzu so dimensioniert, dass die Oberflächenspannung der zur Verabreichung vorgesehenen Infusionsflüssigkeiten in jedem Fall ausreicht, um zu verhindern, dass sich deren Fließfront innerhalb der Infusionsleitung an hierin enthaltenen Luftblasen vorbeischieben kann.

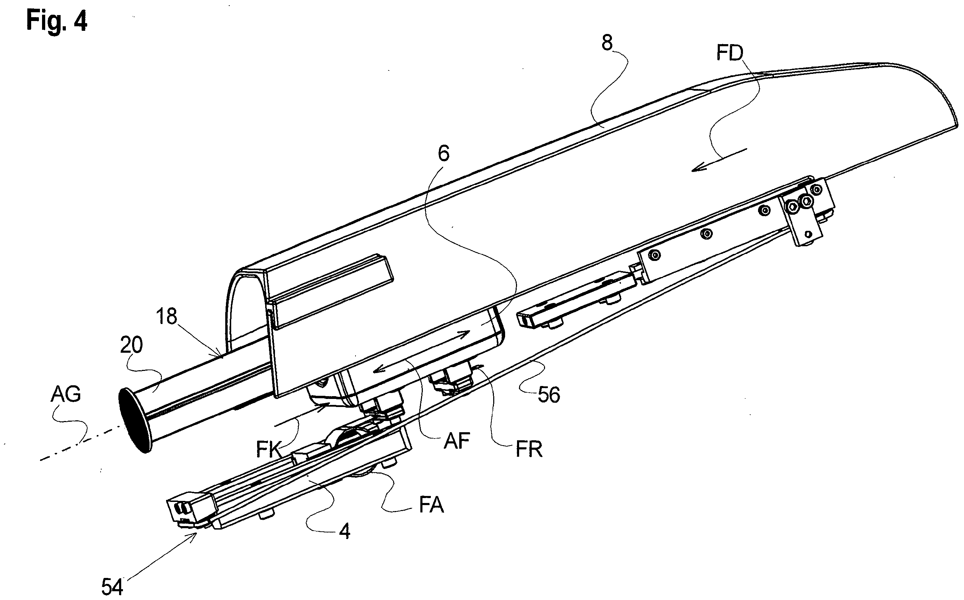

Um die Infusionsgeräteaufnahme 6 mit daran aufgenommenem gefüllten Infusionsgerät 18 selbsttätig in eine Ausgangstellung bringen zu können, in der der Stößel 20 das Stößelanschlags/-betätigungs-Element 24 der Betätigungsvorrichtung 30 kontaktiert und sowohl das Infusionsgerät 18 als auch die Infusionsleitung 38 entlüftet ist, ist eine Verlagerungsvorrichtung 54 vorgesehen, wie aus Fig. 4 zu entnehmen ist. Diese weist einen Seilzug 56 auf, der den Gehäusedeckel 8 mit der Infusionsgeräteaufnahme 6 verbindet. Der Seilzug 56 ist dabei derart am Gehäuse 4 umgelenkt, dass er beim Verlagern des Gehäusedeckels 8 von der Offenstellung in die Schließstellung mittels einer an diesem aufgebrachten Kraft FD die Infusionsgeräteaufnahme 6 mit einer Antriebskraft AF beaufschlagt, die diese entgegen einer Federkraft FK einer Rückstellfeder FR in Richtung des Stößelanschlags/-betätigungs-Elementes 24 verlagert. Der Gehäusedeckel 8 wirkt hierbei selbst als Stellglied der Verlagerungsvorrichtung 54, durch dessen Beaufschlagung die Verlagerung der Infusionsgeräteaufnahmen 6 erfolgt. Alternativ hierzu kann die Verlagerungsvorrichtung 54 auch ein separates Stellglied aufweisen, das entweder durch die Schließbewegung des Gehäusedeckels 8 verlagerbar ist oder das separat zum Gehäusedeckel 8 ausgeführt ist und unabhängig von diesem betätigt werden kann (nicht dargestellt).

Bei einer Verlagerung des Gehäusedeckels 8 aus der Schließstellung in die Offenstellung wird die Infusionsgeräteaufnahme 6 dagegen durch die elastischen Rückstellkräfte der in der Schließstellung vorgespannten Rückstellfeder FR mit der nun entgegen gesetzt gerichteten Antriebskraft AF beaufschlagt, so dass die Infusionsgeräteaufnahme 6 wieder von dem Stößelanschlags/-betätigungs-Element 24 weg bewegt wird.

Ferner ist zwischen dem Seilzug 56 und der Infusionsgeräteaufnahme 6 eine Ausgleichsfeder FA angeordnet. Diese dient dazu, verschiedene Längserstreckungen der eingesetzten Infusionsgeräte 18 entlang ihrer jeweiligen Haupterstreckungsachse AG auszugleichen, die beispielsweise aufgrund unterschiedlicher Füllstände des Behälters 22 ausgebildet werden. Außerdem können durch die Ausgleichsfeder FA Druckspitzen ausgeglichen werden, die beispielsweise bei ruckartigem Verschließen des Gehäusedeckels 8 entstehen können.

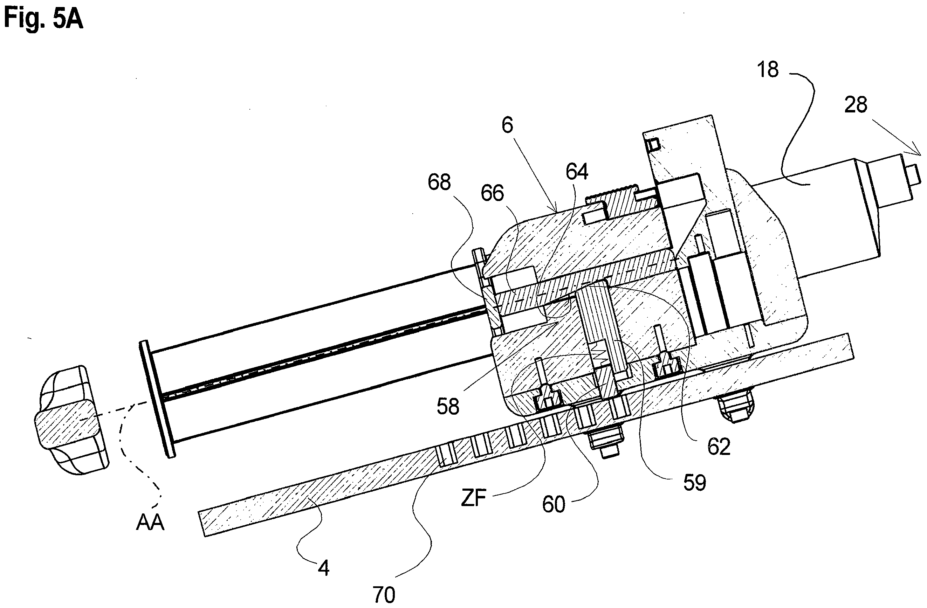

Zur Festlegung der Infusionsgeräteaufnahme 6 mit daran aufgenommenem Infusionsgerät 18 in der entlüfteten Ausgangsstellung ist eine Verriegelungsanordnung vorgesehen, die ein an der Infusionsgeräteaufnahme 6 mitgeführtes Keilgetriebe 58 aufweist, wie es in Fig. 5A dargestellt ist. Dieses weist ein Zwischenelement 59 auf, an dem sich ein Verriegelungselement 60 unter Zwischenlage einer Zwischenfeder ZF abstützt und das an der Infusionsgeräteaufnahme 6 quer zur Haupterstreckungsachse AA verschiebbar gehalten ist.

Dieses Verriegelungselement 60 ist in der Offenstellung des Gehäusedeckels 8, wie dargestellt, zunächst in einer eingerückten Passivstellung angeordnet. In dieser liegt ein rückseitiges Ende 62 des Zwischenelementes 59 an einem rampenförmig ausgeschnittenen Kulissenbereich 64 eines Stellelementes 66 an, das in Richtung der Haupterstreckungsachse AA gegenüber dem Verriegelungselement 60 verschiebbar gelagert ist. An einem äußeren Ende des Stellelementes 66 liegt wiederum ein Spritzenkragen 68 des in der Infusionsgeräteaufnahme 6 aufgenommenen Infusionsgerätes 18 an.

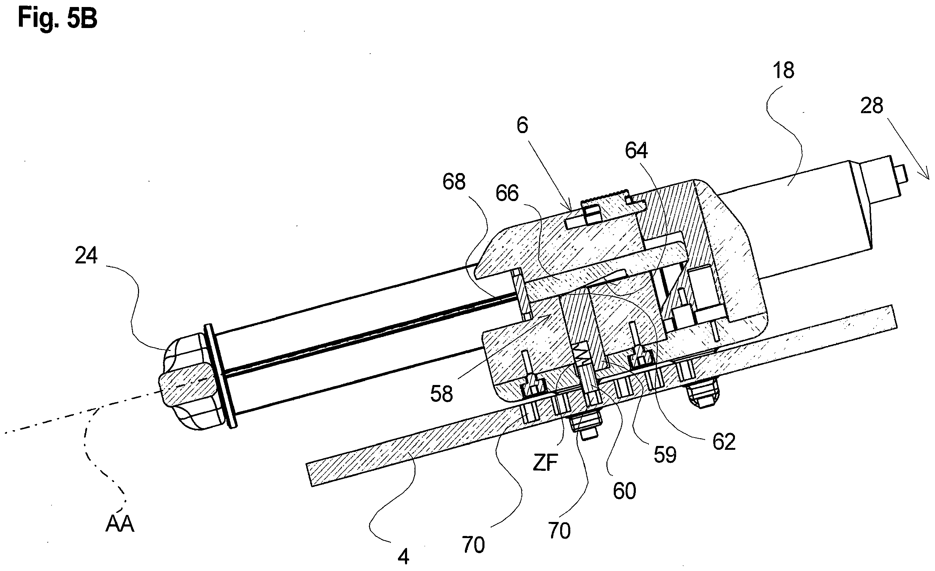

Hierdurch kann das Stellelement 66 bei Beaufschlagung des Stößels 20 durch das Stößelanschfags/-betätigungs-Element 24 über den Spritzenkragen 68 mit einer Kraft beaufschlagt werden, die es in Richtung des auslassseitigen Endes 28 verschiebt. Hierbei drückt das Stellelemente 66 über den Kulissenbereich 64 gegen das rückseitige Ende 62 des Zwischenelementes 59, wodurch dieses nach unten verschoben wird. Hierbei beaufschlagt das Zwischenelement 59 über die Zwischenfeder ZF wiederum das Verriegelungselement 60, das hierbei gegen einen Bereich des Gehäuses 4 vorgespannt wird, in den mehrere Verriegelungsaufnahmen 70 eingelassen sind. Sobald nun das Verriegelungselement 60 bei der Verlagerung der Infusionsgeräteaufnahme 6 genau über einer dieser Verriegelungsaufnahmen 70 positioniert ist, wird es von der dargestellten Passivstellung in eine Aktivstellung verlagert, in der es in die Verriegelungsaufnahme 70 eingreift, wie in Fig. 5B dargestellt.

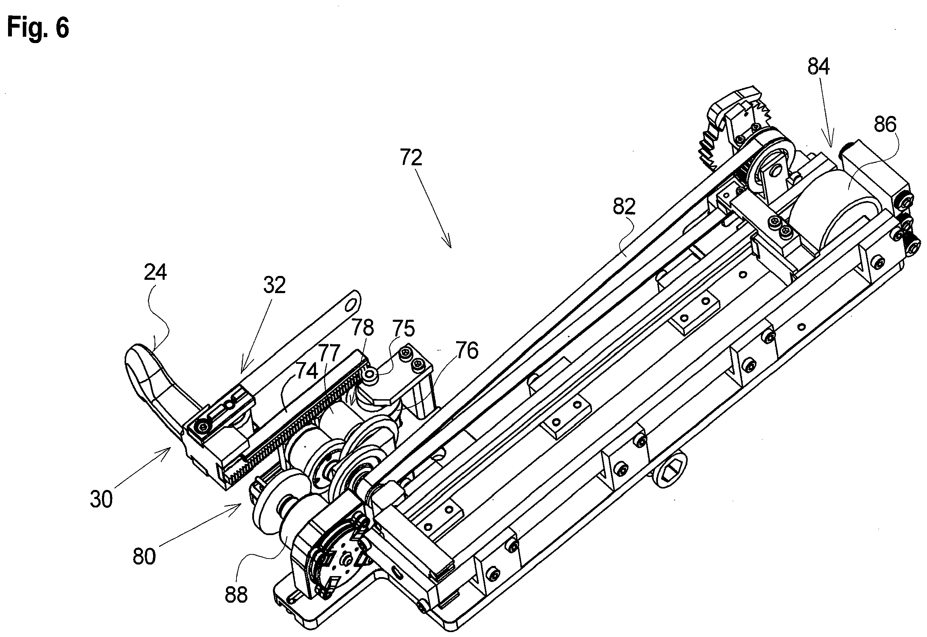

Um nun in dieser verriegelten Ausgangsstellung des Infusionsgerätes 18 die Infusion in einer vorbestimmten Weise durchführen zu können, weist die Dauerinfusionsvorrichtung 2 neben dem eigentlichen Antrieb 32 eine Steuerungsanordnung 72 auf, die in Fig. 6 dargestellt ist. Mittels dieser Steuerungsanordnung 72 kann eine gewünschte Vorschubgeschwindigkeit der Betätigungsvorrichtung 30 beziehungsweise deren Stößelanschlags/-betätigungs-Elementes 24 eingestellt werden.

Hierzu weist die Betätigungsvorrichtung 30 eine Zahnstange 74 auf, die mit einem Zahnrad 75 kämmt, das über eine Welle mit einem Schneckenrad 76 der Steuerungsanordnung verbunden ist. Das Schneckenrad 76 bildet hierbei mit einem Schneckenelement 77 ein selbsthemmendes Schneckenradgetriebe 78, dessen Selbsthemmung durch ein Steuerungsgetriebe 80 aufgehoben werden kann, das mit dem Schneckenelement 77 zusammen wirkt. Das Steuerungsgetriebe 80 kann dabei beispielhaft mittels eines Zahnriemens 82 mit einem Drehmoment beaufschlagt werden, wobei der Zahnriemen 82 wiederum von einem Federkraftspeicher 84 mit einer eigenen Steuerungsantriebsfeder 86 angetrieben ist.

Der eigentliche Antrieb der Betätigungsvorrichtung 30 erfolgt somit allein durch die Antriebsfeder 34, wobei die hierbei wirkende Vorschubgeschwindigkeit des angetriebenen Stößelanschlags/-betätigungs-Elementes 24 davon abhängt, mit welcher Geschwindigkeit das Schneckenradgetriebe 78 dreht.

Zur stufenlosen Einstellung der gewünschten Vorschubgeschwindigkeit ist dabei am Steuerungsgetriebe 80 ein Rotationsdämpfer 88 vorgesehen, der seitens des Benutzers über den zweiten Drehschalter 16 einstellbar ist.

Ein üblicher Infusionsvorgang läuft demnach wie folgt ab:
In der Offenstellung des Gehäusedeckels 8 gemäß Fig. 1 wird das mit dem zu verabreichenden flüssigen Medikament M gefüllte Infusionsgerät 18 in die Infusionsgeräteaufnahme 6 eingesetzt und gemäß den Fig. 2 und 3 mit der Infusionsleitung 38 und der daran vorgesehenen Entlüftungseinrichtung 44 verbunden.

Durch Verbringen des Gehäusedeckels 8 in die Schließstellung wird die Infusionsgeräteaufnahme 6 über den Seilzug 56 mit der Antriebskraft AF beaufschlagt und in Richtung des Stößelanschlags/-betätigungs-Elementes 24 verlagert. Sobald der Stößel 20 des Infusionsgerätes 18 dabei gegen das Stößelanschlags/- betätigungs-Element 24 gedrückt wird, wird das im Behälter 22 enthaltene flüssige Medikament über den Spritzenauslass 40 in die Infusionsleitung 38 transportiert. Die im Behälter 22 und der Infusionsleitung 38 enthaltene Luft wird hierbei zur Entlüftungseinrichtung 44 transportiert und kann hier über die Membrane 46, 48 nach außen entweichen.

Sobald der Stößel 20 jedoch soweit in den Behälter 22 hinein verschoben ist, dass die Luft vollständig entwichen ist und nun das flüssige Medikament M an der ersten hydrophoben Membran 46 ansteht, baut sich über die Flüssigkeit ein Druckwiderstand auf, der der am Stößel 20 wirkenden Bewegungskraft entgegenwirkt, wodurch wiederum das Stellelement 66 des Keilgetriebes 58 (siehe Fig. 5) mit einer Kraft beaufschlagt wird und sich dadurch gegenüber dem Verriegelungselement 60 verlagert. Hierbei drückt der Kulissenbereich 64 gegen das rückseitige Ende 62 und verschiebt dadurch das Zwischenelement 59, wodurch wiederum das Verriegelungselement 60 gegen das Gehäuse 4 vorgespannt wird.

Wenn hierbei trotz vollständig eingeschobenem Stellelement 66 das Verriegelungselement 60 noch immer in keine Verriegelungsaufnahme 70 eingreifen kann, treten an der ersten hydrophoben Membran 46 durch den erhöhten Flüssigkeitsdruck geringfügige Mengen des flüssigen Medikamentes M aus, wodurch sich die Infusionsgeräteaufnahme 6 zumindest geringfügig weiter in Richtung des Stößelanschlags/-betätigungs-Elementes 24 bewegen kann. Diese weitere Verlagerung erfolgt solange, bis das Verriegelungselement 60 in eine der im Gehäuse 4 eingelassenen Verriegelungsaufnahmen 70 eingreift. In dieser Stellung ist die Infusionsgeräteaufnahme 6 mit dem daran aufgenommenen Infusionsgerät 18 gegenüber dem Gehäuse 4 festgelegt und sowohl der Behälter 22 als auch die Infusionsleitung 38 sind entlüftet.

Spätestens in diesem Zustand kann nun über die Drehschalter 14, 16 die zu verabreichende Menge und die Dauer beziehungsweise die Geschwindigkeit des Infusionsvorganges bestimmt werden. Zudem kann nun die Entlüftungseinrichtung 44 entfernt und die Infusionsleitung 38 beispielsweise an einen Katheter angeschlossen werden (nicht dargestellt).

Durch Betätigung des Druckknopfes 10 wird dann der Zahnriemen 82 freigegeben (siehe Fig. 6) der durch die zuvor aufgezogene Steuerungsantriebsfeder 86 angetrieben wird. Dieser treibt wiederum das Steuerungsgetriebe 80 an, das in Abhängigkeit der über den Rotationsdämpfer 88 beziehungsweise über den Drehschalter 16 eingestellten Geschwindigkeit das Schneckenrad 76 mit einem Drehmoment beaufschlagt.

Durch dieses Drehmoment wird die Selbsthemmung des Schneckenradgetriebes 78 aufgehoben, wodurch sich nun auch die Zahnstange 74 der Betätigungsvorrichtung 30 in Abhängigkeit der an der Steuerungsanordnung 72 eingestellten Geschwindigkeit verlagern kann. Der Antrieb der Betätigungsvorrichtung 30 erfolgt dabei durch die zuvor gespannte Antriebsfeder 34, mittels der das Stößelanschlags/-betätigungs-Element 24 gegen den Stößel 20 gedrückt wird.

Da das übrige Infusionsgerät 18 über die verriegelte Infusionsgeräteaufnahme 6 an dem Gehäuse 4 festgelegt ist, wird das in dem Behälter 22 gespeicherte flüssige Medikament M hierbei durch den druckbeaufschlagten Stößel 20 solange in die Infusionsleitung 38 transportiert, bis die an der Steuerungsanordnung eingestellte Infusionsmenge erreicht ist oder der Druckknopf 12 zum Stoppen des Infusionsvorganges gedrückt wird. In beiden Fällen wird hierbei der Antrieb des Steuerungsgetriebes 80 über den Zahnriemen 82 beendet und dadurch die Selbsthemmung am Schneckenradgetriebe 78 wieder hergestellt.

## Patentansprüche

1. Vorrichtung für Dauerinfusionen (2)
mit einem Gehäuse (4), in dem eine Infusionsgeräteaufnahme (6) für die Aufnahme eines Infusionsgerätes (18) mit wenigstens einem mittels eines Stößels (20) auspressbaren Behälters (22) vorgesehen ist, die sich entlang einer Haupterstreckungsachse (AA) von einem stößelseitigen Ende (26) zu einem auslassseitigen Ende (28) erstreckt und
mit einer Betätigungsvorrichtung (30) zum selbsttätigen Auspressen des Infusionsgerätes (18), einem Antrieb (32) zur Verlagerung eines Stößelanschlags/-betätigungs-Elementes (24) der Betätigungsvorrichtung (30) und einer Steuerungsanordnung (72), mittels der eine Vorschubgeschwindigkeit der Betätigungsvorrichtung (30) einstellbar ist,
**dadurch gekennzeichnet, dass** die Haupterstreckungsachse (AA) der Infusionsgeräteaufnahme (6) gegenüber einer Lagerebene (E) des Gehäuses (4) einen vorgegebenen Anstellwinkel (W) aufweist und dabei das auslassseitige Ende (28) oberhalb des stößelseitigen Endes (26) der Infusionsgeräteaufnahme (6) angeordnet ist.

2. Dauerinfusionsvorrichtung nach Anspruch 1, **dadurch gekennzeichnet, dass** der Anstellwinkel (W) zwischen 5 und 90° beträgt.

3. Dauerinfusionsvorrichtung nach Anspruch 1 oder 2, **dadurch gekennzeichnet, dass** die Infusionsgeräteaufnahme (6) eine Codierung für eine vorbestimmte Winkelposition des aufzunehmenden Infusionsgerätes (18) bezüglich der Haupterstreckungsachse (AA) aufweist.

4. Dauerinfusionsvorrichtung nach einem der Ansprüche 1 bis 3, **dadurch gekennzeichnet, dass** die Infusionsgeräteaufnahme (6) mit dem stößelseitigen Ende (26) in Richtung des Stößelanschlags/-betätigungs-Elementes (24) verlagerbar ist und hierzu mit einem zwischen einer Schließstellung und einer Offenstellung verlagerbaren Stellglied bewegungsgekoppelt ist.

5. Dauerinfusionsvorrichtung nach Anspruch 4, **dadurch gekennzeichnet, dass** das Stellglied Teil einer Verlagerungsvorrichtung (54) ist, die bei einer Schließbewegung eines Gehäusedeckels (8) mit einer Antriebskraft (AF) beaufschlagbar ist.

6. Dauerinfusionsvorrichtung nach Anspruch 5, **dadurch gekennzeichnet, dass** die Verlagerungsvorrichtung (54) eine Ausgleichsfeder (F) zum Ausgleich von Toleranzen aufweist.

7. Dauerinfusionsvorrichtung nach einem der Ansprüche 1 bis 6, **dadurch gekennzeichnet, dass** der Antrieb (32) im Wesentlichen durch eine Antriebsfeder (34) gebildet ist.

8. Dauerinfusionsvorrichtung nach einem der Ansprüche 1 bis 7, **dadurch gekennzeichnet, dass** die Steuerungsanordnung (72) eine Steuerungsantriebsfeder (86) aufweist.

9. Dauerinfusionsvorrichtung nach Anspruch 7 oder 8, **dadurch gekennzeichnet, dass** zwischen dem Antrieb (32) und der Steuerungsanordnung (72) eine Selbsthemmung vorgesehen ist, die mittels eines seitens der Steuerungsanordnung (72) generierbaren Drehmomentes überwindbar ist.

10. Dauerinfusionsvorrichtung nach Anspruch 9, **dadurch gekennzeichnet, dass** die Selbsthemmung durch ein Schneckenradgetriebe (78) gebildet ist.

11. Dauerinfusionsvorrichtung nach einem der Ansprüche 1 bis 10, **dadurch gekennzeichnet, dass** die Steuerungsanordnung (72) einen einstellbaren Rotationsdämpfer (88) aufweist, mittels dem die Vorschubgeschwindigkeit der Betätigungsvorrichtung (30) begrenzbar ist.

12. Infusionsanordnung mit einer Dauerinfusionsvorrichtung nach einem der Ansprüche 1 bis 11 und einem Infusionsgerät, **dadurch gekennzeichnet, dass** das Infusionsgerät (18) durch eine in Größe und Geometrie auf die Infusionsgeräteaufnahme (6) abgestimmte Infusionsspritze mit einem exzentrisch angeordneten Spritzenauslass (40) gebildet ist.

13. Infusionsanordnung nach Anspruch 12, **dadurch gekennzeichnet, dass** die Infusionsspritze mit einer Infusionsleitung (38) verbunden ist, an deren Ende eine zur Entlüftung dienende hydrophobe Membran (46; 48) vorgesehen ist, die luftdurchlässig ist aber flüssigkeitsabdichtend wirkt.

14. Infusionsanordnung nach Anspruch 13, **dadurch gekennzeichnet, dass** die in der Infusionsgeräteaufnahme (6) aufgenommene Infusionsspritze durch die Schließbewegung des Gehäusedeckels (8) oder der Verlagerungsvorrichtung (54) soweit gegen das Stößelanschlags/-betätigungs-Element (24) verlagerbar ist, dass die hydrophobe Membran (46; 48) durch die im Behälter (22) aufgenommene Infusionsflüssigkeit über die Infusionsleitung (38) beaufschlagbar ist.

15. Infusionsanordnung nach Anspruch 13 oder 14, **dadurch gekennzeichnet, dass** der hydrophoben Membran (46; 48) eine weitere hydrophobe Membran (48; 46) vorgeschaltet ist, die ab einem vorbestimmten Schwellendruck hinsichtlich der Infusionsflüssigkeit durchlässig ist.

16. Infusionsanordnung nach einem der Ansprüche 12 bis 15, **dadurch gekennzeichnet, dass** eine Verriegelungsanordnung vorgesehen ist, mittels der die Infusionsgeräteaufnahme (6) gegenüber dem Gehäuse (4) festlegbar ist, wobei die Verriegelungsanordnung durch einen an der Infusionsgeräteaufnahme (6) angreifenden Bewegungswiderstand aktivierbar ist, der bei der Verlagerung des Infusionsgerätes (18) gegen das Stößelanschlags/-betätigungs-Element (24) durch den sich zwischen der hydrophoben Membran (46; 48) und dem Spritzenstößel aufbauenden Flüssigkeitsdruck generierbar ist.

17. Infusionsanordnung nach Anspruch 16, **dadurch gekennzeichnet, dass** die Verriegelungsanordnung ein Keilgetriebe (58) aufweist, mittels dem wenigstens ein an der Infusionsgeräteaufnahme (6) mitgeführtes Verriegelungselement (60) in eine gehäuseseitige Verriegelungsaufnahme (70) verbringbar ist.

## Claims

1. Continuous infusion device (2)
comprising a housing (4), in which an infusion appliance seat (6) is provided for receiving an infusion appliance (18) with at least one container (22) which can be pressed empty by means of a ram (20), which infusion appliance seat extends along a main axis of extent (AA) from a ram-side end (26) to an outlet-side end (28), and
with an actuator (30) for automatically emptying the infusion appliance (18), a drive (32) for moving a ram abutment/actuation element (24) of the actuator (30), and a control arrangement (72), by means of which a speed of advance of the actuator (30) is adjustable,
**characterised in that** the main axis of extent (AA) of the infusion appliance seat (6) exhibits a predetermined relief angle (W) in relation to a support plane (E) of the housing (4), and here the outlet-side end (28) is arranged above the ram-side end (26) infusion appliance seat (6).

2. Continuous infusion device according to claim 1, **characterised in that** the relief angle (W) is between 5 and 90°.

3. Continuous infusion device according to claim 1 or 2, **characterised in that** the infusion appliance seat (6) exhibits a coding for a predetermined angle position of the infusion device (18) which is to be received in relation to the main axis of extent (AA).

4. Continuous infusion device according to any one of claims 1 to 3, **characterised in that** the infusion appliance seat (6) can be displaced with the ram-side end (26) in the direction of the ram abutment/actuation element (24), and for this purpose is movement-coupled to an actuator element which can be moved between a closed position and an open position.

5. Continuous infusion device according to claim 4, **characterised in that** the actuator element is a part of a displacement device (54), which, at a closure movement of the housing cover (8), can be subjected to a drive force (AF).

6. Continuous infusion device according to claim 5, **characterised in that** the displacement device (54) comprises a compensation spring (F) for the compensation of tolerances.

7. Continuous infusion device according to any one of claims 1 to 6, **characterised in that** the drive (32) is formed essentially by a drive spring (34).

8. Continuous infusion device according to any one of claims 1 to 7, **characterised in that** the control arrangement (72) comprises a control drive spring (86).

9. Continuous infusion device according to claim 7 or 8, **characterised in that** a self-inhibition arrangement is provided between the drive (32) and the control arrangement (72), which can be overcome by means of a torque which can be generated by the control arrangement (72).

10. Continuous infusion device according to claim 9, **characterised in that** the self-inhibition arrangement is formed by a worm gear drive (78).

11. Continuous infusion device according to any one of claims 1 to 10, **characterised in that** the control arrangement (72) comprises an adjustable rotation damper (88), by means of which the advance speed of the actuator (30) can be limited.

12. Infusion arrangement with a continuous infusion device according to any one of claims 1 to 11, **characterised in that** the infusion device (18) is formed by an infusion needle which is matched in size and geometry to the infusion appliance seat (6), and with an eccentrically-arranged needle outlet (40).

13. Infusion arrangement according to claim 12, **characterised in that** the infusion needle is connected to an infusion line (38), provided at the end of which is a hydrophobic membrane (46; 48) serving to bleed off air, which has an air-permeable effect but has the effect of sealing against fluid.

14. Infusion arrangement according to claim 13, **characterised in that** the infusion needle received in the infusion appliance seat (6) can be displaced by the closure movement of the housing cover (8) or the displacement device (54) sufficiently far against the ram abutment/actuation element (24), that the hydrophobic membrane (46; 48) can be subjected to an infusion fluid, received in the container (22) via the infusion line (38).

15. Infusion arrangement according to claim 13 or 14, **characterised in that** the hydrophobic membrane (46; 48) is arranged upstream of a further hydrophobic membrane (48; 46), which as from a predetermined threshold pressure is permeable in respect of the infusion fluid.

16. Infusion arrangement according to any one of claims 12 to 15, **characterised in that** a locking arrangement is provided, by means of which the infusion appliance seat (6) can be secured in relation to the housing (4), wherein the locking arrangement can be activated by a movement resistance engaging at the infusion appliance seat (6), which, at the displacement of the infusion appliance (18) against the ram abutment/actuation element (24), can be generated by the fluid pressure building up between the hydrophobic membrane (46; 48) and the injection ram,.

17. Infusion arrangement according to claim 16, **characterised in that** the locking arrangement comprises a wedge gear (58), by means of which at least one locking element (60), conveyed together with the infusion appliance seat (6), can be brought into a housing-side locking receiver (70).

## Revendications

1. Dispositif de perfusion continue (2)
comprenant un boîtier (4) dans lequel il est prévu un logement (6) d'appareil de perfusion pour la réception d'un appareil de perfusion (18) comprenant au moins un récipient (22) pouvant être pressé au moyen d'un poussoir (20), le logement s'étendant le long d'un axe d'extension principale (AA) depuis une extrémité côté poussoir (26) à une extrémité côté sortie (28) et
comprenant un dispositif de manoeuvre (30) pour le pressage automatique de l'appareil de perfusion (18), un entraînement (32) pour déplacer un élément (24) de manoeuvre/butée du poussoir du dispositif de manoeuvre (30) et un agencement de commande (72) au moyen duquel une vitesse d'avance du dispositif de manoeuvre (30) est réglable,
**caractérisé en ce que** l'axe d'extension principale (AA) du logement (6) d'appareil de perfusion présente un angle d'incidence (W) prédéterminé par rapport à un plan de support (E) du boîtier (4) et ainsi l'extrémité côté sortie (28) est agencée au-dessus de l'extrémité côté poussoir (26) du logement (6) d'appareil de perfusion.

2. Dispositif de perfusion continue selon la revendication 1, **caractérisé en ce que** l'angle d'incidence (W) est compris entre 5 et 90°.

3. Dispositif de perfusion continue selon la revendication 1 ou 2, **caractérisé en ce que** le logement (6) d'appareil de perfusion comprend un codage pour une position angulaire prédéfinie de l'appareil de perfusion (18) à recevoir par rapport à l'axe d'extension principale (AA).

4. Dispositif de perfusion continue selon l'une des revendications 1 à 3, **caractérisé en ce que** par l'extrémité côté poussoir (26), le logement (6) d'appareil de perfusion est déplaçable en direction de l'élément (24) de manoeuvre/butée du poussoir, et à cet effet est couplé en mouvement avec un organe de réglage déplaçable entre une position de fermeture et une position d'ouverture.

5. Dispositif de perfusion continue selon la revendication 4, **caractérisé en ce que** l'organe de réglage est une partie d'un dispositif de déplacement (54) sur lequel peut s'exercer une force d'entraînement (AF) lors d'un mouvement de fermeture d'un couvercle (8) de boîtier.

6. Dispositif de perfusion continue selon la revendication 5, **caractérisé en ce que** le dispositif de déplacement (54) comprend un ressort de compensation (F) pour compenser des tolérances.

7. Dispositif de perfusion continue selon l'une des revendications 1 à 6, **caractérisé en ce que** l'entraînement (32) est formé essentiellement par un ressort d'entraînement (34).

8. Dispositif de perfusion continue selon l'une des revendications 1 à 7, **caractérisé en ce que** l'agencement de commande (72) comprend un ressort d'entraînement de commande (86).

9. Dispositif de perfusion continue selon la revendication 7 ou 8, **caractérisé en ce qu'**il est prévu entre l'entraînement (32) et l'agencement de commande (72) un auto-blocage surmontable au moyen d'un couple pouvant être généré par l'agencement de commande (72).

10. Dispositif de perfusion continue selon la revendication 9, **caractérisé en ce que** l'auto-blocage est formé par une transmission à vis (78).

11. Dispositif de perfusion continue selon l'une des revendications 1 à 10, **caractérisé en ce que** l'agencement de commande (72) comprend un amortisseur rotatif (88) réglable, au moyen duquel la vitesse d'avance du dispositif de manoeuvre (30) peut être limitée.

12. Agencement de perfusion comprenant un dispositif de perfusion continue selon l'une des revendications 1 à 11 et un appareil de perfusion, **caractérisé en ce que** l'appareil de perfusion (18) est formé par une seringue de perfusion comprenant une sortie de seringue (40) agencée de manière excentrique, la seringue étant adaptée en taille et en géométrie au logement (6) d'appareil de perfusion.

13. Agencement de perfusion selon la revendication 12, **caractérisé en ce que** la seringue de perfusion est reliée à un conduit de perfusion (38) dont l'extrémité comprend une membrane hydrophobe (46 ; 48) servant pour la purge de l'air, qui est perméable à l'air mais étanche au liquide.

14. Agencement de perfusion selon la revendication 13, **caractérisé en ce que** la seringue de perfusion, reçue dans le logement (6) d'appareil de perfusion, est déplaçable par le mouvement de fermeture du couvercle du boîtier (8) ou le dispositif de déplacement (54) contre l'élément (24) de manoeuvre/butée du poussoir, jusqu'à un point où la membrane hydrophobe (46 ; 48) peut être en contact avec le liquide de perfusion contenu dans le récipient (22) par l'intermédiaire du conduit de perfusion (38).

15. Agencement de perfusion selon la revendication 13 ou 14, **caractérisé en ce que** la membrane hydrophobe (46 ; 48) est précédée d'une autre membrane hydrophobe (48 ; 46), qui est perméable au liquide de perfusion à partir d'une pression de seuil prédéfinie.

16. Agencement de perfusion selon l'une des revendications 12 à 15, **caractérisé en ce qu'**il est prévu un agencement de verrouillage, au moyen duquel le logement (6) d'appareil de perfusion est fixable par rapport au boîtier (4), l'agencement de verrouillage étant activable par une résistance au mouvement venant en prise avec le logement (6) d'appareil de perfusion, qui peut être générée par la pression de liquide qui s'établit entre la membrane hydrophobe (46 ; 48) et le poussoir de seringue lors du déplacement de l'appareil de perfusion (18) contre l'élément (24) de manoeuvre/butée du poussoir.

17. Agencement de perfusion selon la revendication 16, **caractérisé en ce que** l'agencement de verrouillage comprend une transmission à clavette (58) au moyen de laquelle au moins un élément de verrouillage (60) entraîné avec le logement (6) d'appareil de perfusion est introductible dans un logement de verrouillage (70) côté boîtier.
